# EUROPEAN PATENT APPLICATION

(11) **EP 4 328 175 A1**
(43) Date of publication of application: **28.02.2024**
(21) Application number: 22208351.1
(22) Date of filing: 18.11.2022
(51) Int. Cl.: B82Y 35/00, C12Q 1/37, C12Q 1/6816, C12Q 1/6869, G01N 33/543

(54) **METHOD FOR SEQUENCING**

(30) Priority: 23.08.2022 EP 22191690; 23.08.2022 EP 22191689
(71) Applicant: Leica Microsystems CMS GmbH, 35578 Wetzlar (DE)
(72) Inventor: ALSHEIMER, Soeren, 60320 Frankfurt (DE)
(74) Representative: Schaumburg und Partner Patentanwälte mbB

(57) **Abstract**

A method is provided for sequencing of target genetic sequences (910) of a plurality of cells (700, 908) comprising the following steps: providing droplets (702, 906), each droplet including at least one cell (700, 908) of the plurality of cells and at least one optically detectable marker (300, 302, 304, 306, 308, 600, 704, 706, 904) comprising: a nucleic acid backbone (100, 102, 104, 106, 108, 314, 601) with a plurality of attachment sites (316, 400, 410) at predetermined positions; a plurality of labels (200, 318, 320, 322, 604, 606, 608, 902) for attachment to at least some of the attachment sites; at least a first orientation indicator and a second orientation indicator; wherein each label comprises at least one dye (202a, 202b, 202c, 202d, 202e, 408, 418), an encoding oligonucleotide portion (404, 414) configured to encode characteristics of the at least one dye, and an attachment oligonucleotide portion (402, 412) configured to reversibly attach to one of the attachment sites (316, 400, 410); and wherein the attachment oligonucleotide portion (402, 412) of each label comprises a unique oligonucleotide sequence configured to bind to a complementary sequence of one of the attachment sites (316, 400, 410). The method further comprising the steps: releasing the target genetic sequences (910) from the cells (700, 908); ligating the target genetic sequences to the labels of the marker to generate sequencing constructs (922, 1000); and sequencing the sequencing constructs.

## Description

### Technical field

The invention relates to a method for sequencing of target genetic sequences of a plurality of cells.

### Background

Rare cells, like e.g. adult stem cells, circulating tumour cells and reactive immune cells (e.g. T-Cells, B-Cells, or NK-cells reactive to a certain antigen), are of great interest to basic and translational researchers. Reactive immune cells such as B-cell clones for instance that react to a certain pathogen, e.g. a virus, and can produce antibodies against a particular pathogen are of great value to generate urgently needed therapeutic antibodies. Similarly, reactive T-cells are sought after in the context of personalised medicine and the treatment of cancer and other diseases. Once a reactive T-cell is identified and isolated, the genetic sequence encoding the corresponding T-cell receptor displaying affinity against the target antigen can be cloned and used to generate genetically engineered T-cells such as CAR-T cells. Similarly, circulating tumour cells are expected to have great value for diagnosing cancer, predicting outcomes, managing therapies, and for the discovery of new cancer drugs and cell-based therapeutics. Suspensions of cells containing rare cells are typically derived from either a tissue sample by means of dissociation or from a liquid biopsy. The identification, analysis, and isolation of rare cells in these samples, particularly the analysis of these cells on the single cell level (single cell analysis, SCA) is therefore of great value for basic and translational research, diagnostic and therapeutic applications as well as in the context of bioprocessing and development and manufacturing of biologics and cellular therapeutics.

As the ability to identify and differentiate diverse cell types expands, the identification of cell types becomes more granular, i.e. the rare cell populations of interest are smaller and better defined. Thus, in order to find rare cells of interest a high (< 100k), very high (< 1M), or ultra-high (> 1M) number of cells typically needs to be analysed.

Specifically, substantial interest exists in technologies that allow the screening of large populations of B-cell or T-cell clones to find clones that exhibit desirable properties. In many cases following the identification of such a clone the corresponding genomic sequence encoding the variable portions of the antibody or TCR is cloned and sequenced. Various methods have been developed to screen single cells or clones and are in use to screen B-/T-cell clones. These include limiting dilution and manual screening in microplates, nano well imaging approaches, optofluidic systems and other approaches based on microfluidics and imaging in flow-through.

While some of these methods are inherently very limited in terms of throughput all of them have a common key limitation in that cloning of a genomic sequence from a clone of interest requires the physical separation of this clone from other clones. This means that either all single cells/clones have to be arrayed at some point in the workflow into wells, nano wells, nano pens or the like, or they have to be sorted from the rest of the cells. It is estimated, however, that one would ideally screen on the order of 10,000,000 million clones per desired antibody or TCR, as this number appears to be in good relation to the actual diversity found in the immune repertoire. Sorting or arraying 10,000,000 million clones per project is prohibitively labour intensive, which is why currently most screening campaigns sample significantly less clones.

Therefore, it is desirable to be able to keep track of individual cells and assign analysis data from different types of analyses to a particular cell from a large number of pooled cells.

### Summary

It is therefore an object to provide a method that enables sequencing target genetic sequences of a large number of individual cells with high-throughput.

The aforementioned object is achieved by the subject-matter of the independent claim. Advantageous embodiments are defined in the dependent claims and the following description.

A method is provided for sequencing of target genetic sequences of a plurality of cells comprising the following steps: providing droplets, each droplet including at least one cell of the plurality of cells and at least one optically detectable marker. The marker comprises a nucleic acid backbone with a plurality of attachment sites at predetermined positions; a plurality of labels for attachment to at least some of the attachment sites; at least a first orientation indicator and a second orientation indicator; wherein each label comprises at least one dye, in particular a fluorescent dye; an encoding oligonucleotide portion configured to uniquely encode characteristics of the at least one dye; and an attachment oligonucleotide portion configured to reversibly attach to one of the attachment sites; and the attachment oligonucleotide portion of each label comprises a unique oligonucleotide sequence configured to bind to a complementary sequence of one of the attachment sites. The method further comprises the steps: releasing the target genetic sequences from the cells; ligating the target genetic sequences to the labels of the marker to generate sequencing constructs; and sequencing the sequencing constructs.

In particular, when ligating the target genetic sequences to the labels of the marker, the target genetic sequences are ligated to the oligonucleotides of the labels, in particular to the encoding oligonucleotide portion and the attachment oligonucleotide portion of the labels of the marker. Thus, the sequencing constructs each comprise at least one of the target genetic sequences and the encoding oligonucleotide portion and the attachment oligonucleotide portion of one of the labels of the marker.

The arrangement of dyes or combinations of dyes on scaffolds made using structural DNA nanotechnology may serve the purpose that with a limited number of dyes by means of combinatorial encoding a large number of combinations of dyes can be generated. The combinatorial encoding may then be combined with spatial encoding on the DNA nanostructure backbone, which allows the precise arrangement of dyes or combinations of dyes on the backbone. This arrangement can be read optically by reading the labels and orientation indicators as well as using DNA sequencing by reading/sequencing the attachment oligonucleotide portion-encoding oligonucleotide portion sequence stretches that link the respective dyes to the respective marker. In this way a large plurality of markers can be generated that (A) allow the efficient particle indexing (identification) of a large number of droplets, (B) allow the optical reading of the index, (C) allow the DNA sequencing-based reading of the index, and (D) allow the physical linkage of parts of the index or the entire index (concatenate of all attachment oligonucleotide portion-encoding oligonucleotide portion sequence stretches of a given marker required to identify given marker reliably) to genetic target sequences of interest, which in turn (E) enables the sequencing of particle index-genetic target sequence-fusion products, which (F) enables that the genetic target sequence(s) of interest stemming from clones of interest can be retrieved easily by sequencing the concatenates. Clones of interest can in this case be easily identified using imaging-based screening and assays for e.g. antigen binding, antibody aggregation, clone-specific productivity, cytokine secretion, activity of reporters in target cells, killing assays, growth kinetic assays etc. The particle index provided by the marker in particular allows the faithful recognition of the same clone during the imaging as well as the reliable orientation in 3D from one time point to the next in time series acquisitions or sequential assays. In this way a large number of clones can be qualified and ranked according to multiple relevant criteria. The method further allows that target genetic sequences of interest from these prioritized clones may be efficiently retrieved. At this point two alternative example methods may be provided. The first is based on ligating a part of the sequence stretches necessary to read the full index to a given target genetic sequence to give the sequencing construct. In this case the full index is read by sequencing a sufficient number of ligates to read the full index/code and sequencing has to be performed on the single droplet level.

In a second alternative example method, complete concatenates between all encoding oligonucleotide portion-attachment oligonucleotide portion-sequence stretches belonging to a given marker in a given droplet are ligate together (concatenated) with the target genetic sequence(s) to give the sequencing construct.

Such target genetic sequences may be a single or multiple sequences. In a particular preferred embodiment, the target genetic sequences encode antibodies or T-cell receptors, or portions thereof for example regions encoding the framework, variable region, hypervariable region, complementary determining regions.

Details of suitable markers are disclosed in the patent application with the application number EP22153210.4, the content thereof is incorporated herein by reference.

The orientation indicators are configured to attach to the backbone and may be fluorescent dyes, for example. The orientation indicators may be used to visually determine the orientation of the marker in space. The encoding oligonucleotide portion is a unique sequence of nucleic acids that are unique to the excitation/emission wavelength and/or the fluorescent lifetime of the at least one dye of the label. The marker is thus visually unambiguously identifiable by its unique combination of dyes in each label as well as their specific attachment sites. The same marker is unambiguously identifiable by sequencing the unique encoding oligonucleotide portions and the attachment oligonucleotide portions.

By ligating the target genetic sequences to the oligonucleotides of the labels, in particular the to the encoding oligonucleotide portion and the attachment oligonucleotides portion of the labels of the marker, the target genetic sequences are physically linked to the sequence information that enables unambiguously identifying the marker and therefore to linking the target genetic sequences to the marker.

Preferably, the nucleic acid backbone comprises scaffold strands, and staple strands configured to bind to the scaffold strands at predetermined positions to fold the scaffold strand into a predetermined shape. The backbone may be a DNA-origami. These DNA origami structures may range in size from a few nanometres into the micron range. For the fabrication of such DNA origami-based structures longer DNA molecules (scaffold strands) are folded at precisely identified positions by so called staple strands. The DNA origami may be designed to provide a self-assembly backbone of a particular predetermined shape. This enables an easy and reproducible synthesis and assembly of the backbone. Staple strands may be position-selectively functionalised. The positional resolution in this case is limited by the size of a nucleotide, which is in the range of a nanometre or below. This has been exploited in the prior art to generate fluorescent standards, wherein fluorescent dyes are connected to precisely located bands on the DNA origami. These standards are known as "nanoruler" and are used for the calibration of imaging systems like confocal or super resolution microscopes (e.g. STED), for example, as disclosed by US2014/0057805 A1.

The DNA origami provides a scaffold for the labels. Preferably, the DNA origami structure comprises at least one scaffold strand and multiple staple strands, wherein the staple strands are complementary to at least parts of the scaffold strand and configured to bring the scaffold strand into a predetermined conformation. In particular, the strands are oligonucleotides. This enables generating backbones with predetermined two- or three-dimensional shapes that can self-assemble. Further, this enables the site-specific placement of attachment sites on the backbone.

The attachment sites being unique nucleic acid sequences, preferably of the staple strands. Preferably, the labels may be attached to staple strands of the backbone at predetermined attachment sites. Since the staple strands are located at predetermined positions the positions of the attachment sites may equally be predetermined. Thus, the attachment site (of the nucleic acid backbone) is a unique oligonucleotide sequence complementary to the attachment oligonucleotide portion of one label.

Alternatively, the nucleic acid backbone may comprise or consist of DNA bricks. These DNA bricks are shorter length oligonucleotides that have overlapping hybridising stretches in order to bind to each other and assemble the backbone. In this case, the oligonucleotides of the labels may form an integral part of the nucleic acid backbone. Binding to attachment sites on the shorter length oligonucleotides of the DNA bricks in order to generate the nucleic acid backbone.

Preferably, the labels are released from the nucleic acid backbone of the marker prior to sequencing, in a particular alternative prior to ligating or prior to amplification. This enables particularly robust sequencing.

Preferably, the target genetic sequences and the oligonucleotide portions of the labels are amplified prior to sequencing, in particular by adding primers and carrying out PCR. This step is in particularly carried out after releasing the target genetic sequence. The amplification may further include introducing restriction or ligation sites in the amplification product. This enables particularly robust sequencing. Particularly preferred is that the label of the marker comprises a cleavage site configured to separate the dye from the oligonucleotide of the label and wherein prior to ligating, or alternatively prior to amplifying, the dye of each label is cleaved off the oligonucleotide of the label at the cleavage site. This enables particularly robust sequencing.

Preferably, the droplet is a liquid droplet, in particular with a discrete volume, of a first liquid (or first fluid) in an immiscible second liquid (or second fluid) or a solid droplet. This enables the processing the plurality of cells in the same volume whilst keeping the marker in association with the respective cell within the droplet.

The liquid droplet may be based on water-oil emulsion droplet technology. Examples of this include microfluidicly generated droplets such as Bio-Rad droplet digital PCR technology.

The solid droplet may comprise a polymeric compound, in particular a hydrogel. This enables particularly easy handling of the cell with the markers in the droplet. For further examples of a hydrogel droplet or beads comprising a cell and a generalised method for imaging a droplet comprising a cell, reference is made to the applications PCT/EP2021/058785 and PCT/EP2021/061754, the content of which are full incorporated herein by reference.

Further, the document WO 2019/028166 A1 discloses hydrogel beads suitable for use with sequencing of genetic sequences within the bead.

Preferably, the step of providing droplets includes embedding cells and markers in droplets, preferably by means of a microfluidic device. This enables particularly efficient embedding of cells and markers in the droplets.

Preferably, the cells are cultured in the droplets, in particular prior to releasing the target genetic sequences from the cells. This enables analysing cells over or after a period of time.

Preferably, an optical readout of at least one of the droplets with the at least one cell and the at least one marker is acquired. In particular, this is acquired prior to releasing the target genetic sequences. This enables visually analysing the cell within the droplet and identifying the marker.

The optical readout may be an image-based readout, which may be acquired on a microscope like a point-scanning confocal or a camera-based/widefield imaging system for example a spinning disk microscope, a light sheet fluorescence microscope, a light field microscope, a stereomicroscope. Further the optical readout may be non-image-based readouts for example in a cytometer or a flow-through based readout device with at least one-point detector or a line detector. A readout may consist of a discrete readout, for example a single acquisition of an emission spectrum or image stack, a readout may be a readout data stream, for example in a point-scanning confocal or cytometer, which is substantially continuous. Further a readout may be a sequence of images for example a spectral or hyperspectral image stack, wherein in each image fluorescence emission of different wavelength bands is recorded.

The optical readout may be generated by a readout device used to perform fluorescence multi-colour reading or imaging. The readout device typically includes at least one excitation light source, a detection system including at least one detection channel and may further contain filters and/or dispersive optical elements such as prisms and/or gratings to route excitation light to the sample and/or to route emission light from the sample onto to a detector or onto an appropriate area of the detector. The detection system may comprise several detection channels, may be a spectral detector detecting multiple bands of the spectrum in parallel, or a hyperspectral detector detecting a contiguous part of the spectrum. The detection system contains at least one detector, which may be a point-detector (e.g. a photomultiplier, an avalanche diode, a hybrid detector), an array-detector, a camera, hyperspectral camera. The detection system may record intensities per channel as is typically the case in cytometers or may be an imaging detection system that records images as in the case of plate readers or microscopes. A readout device with one detector channel, for example a camera or a photomultiplier, may generate readouts with multiple detection channels using, for example, different excitation and emission bands.

Particularly preferred is that in the optical readout of the at least one of the droplets, the at least one marker in the droplet is determined based on the at least one dye of each of the labels of the marker. This enables identification of the marker based on the dyes of the marker.

Preferably, in sequencing data generated during sequencing of the sequencing constructs of the at least one of the droplets, the sequences of the attachment oligonucleotide portion and the encoding oligonucleotide portion are determined and wherein the sequencing data of the at least one droplet is correlated with the optical readout of the at least one droplet based on the presence of the sequences in the sequencing data and the marker present in the at least one droplet. This enables linking the optical readout to the sequencing data efficiently. Thus, phenotypes identified in the optical readout may be linked to particular genotype identified in the sequencing data, for example.

Preferably, the target genetic sequences are generated by reverse transcription of mRNA of the at least one cell. This enables sequencing of particularly diverse target genetic sequences.

Preferably, the plurality of cells are immunological cells and wherein the target genetic sequences are immunoglobulin genetic sequences, in particular VDJ sequences. This enables identifying immunoglobulin genetic sequences of a large number of immunological cells efficiently, for example.

Preferably, each droplet further comprises at least one non-immunological cell and wherein the at least one immunological cell is specific to antigens of the non-immunological cell. This enables efficiently identifying immunoglobin genetic sequences specific to antigens of the non-immunological cell.

Preferably, the step of ligating includes ligating together all target genetic sequences of the cell of one of the droplets to the labels of the marker of one of the droplets to generate the sequencing constructs. In particular, all target genetic sequences are ligated to the oligonucleotides of the labels of the marker, in particular to the encoding oligonucleotide portion and the attachment oligonucleotide portion of the labels of the marker. This enables robust generation of the sequencing constructs. Further, this enables particularly efficient sequencing of the sequencing constructs without the need to separate the individual droplets before sequencing. In particular, this enables particularly robust correlation of sequencing data of the at least one droplet with the optical readout of the at least one droplet.

Preferably, the sequencing constructs of all droplets are pooled and sequenced together. This enables particularly efficient sequencing of the sequencing constructs without the need to separate the individual droplets before sequencing.

### Short Description of the Figures

Hereinafter, specific embodiments are described referring to the drawings, wherein:
- Fig. 1: shows several nucleic acid backbones with orientation indicators,
- Fig. 2: shows an example of a label for attachment to attachment sites of the backbones,
- Fig. 3: shows a variety of rod-shaped markers,
- Fig. 4: shows details of a rod-shaped marker,
- Fig. 5: shows steps for preparing a label for sequencing,
- Fig. 6: shows a cube-shaped marker,
- Fig. 7: shows a droplet with a cell and markers,
- Fig. 8: shows a flow chart of a method for sequencing of target genetic sequences,
- Fig. 9: shows details of steps to generate random sequencing constructs,
- Fig. 10: shows details of steps to generate predetermined sequencing constructs;
- Fig. 11: shows a hypothetical example of a database entry for an optically detectable marker; and
- Fig. 12: illustrates by way of example the amount of different possible combinatorial markers by using 10 different fluorescent dyes for a rod-shaped marker.

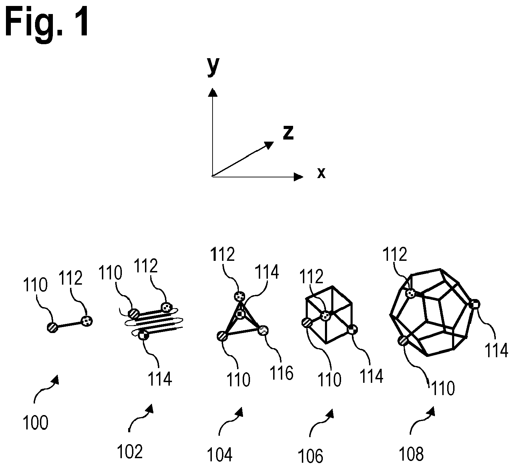

Figure 1 shows schematically nucleic acid backbones 100, 102, 104, 106, 108 with different geometries. Generally, the backbones 100, 102, 104, 106, 108 comprise nucleic acids. In particular, the backbones 100, 102, 104, 106, 108 are DNA-origami based, which allows generating predetermined, stable two- and three-dimensional shapes. Further, this allows generating a plurality of attachment sites at predetermined positions along the backbones 100, 102, 104, 106, 108. The attachment sites are stretches of oligonucleotide, that are unique and allow the hybridisation of complementary oligonucleotides, for example to attach labels to the backbones 100, 102, 104, 106, 108 at these predetermined positions.

Backbone 100 is linear or rod-like. It comprises a first orientation indicator 110 and a second orientation indicator 112. The orientation indicators 110, 112 may be used to determine the orientation, directionality or polarity of the backbone 100. The orientation indicators 110, 112 may comprise a dye, in particular a fluorescent dye, such as fluorescein or a fluorescent protein. In addition, the dye of the first orientation indicator 110 has different characteristics than the dye of the second orientation indicator 112. The characteristics may include fluorescent emission characteristics, excitation characteristics or lifetime characteristics. This enables differentiating between the first and the second orientation indicators 110, 112 in an optical readout of the backbone 100, for example generated by a microscope, a cytometer, or an imaging cytometer. The orientation indicators 110, 112 are arranged spaced apart from each other. Preferably each orientation indicator 110, 112 is arranged on the backbone 100 at opposite ends. Thus, the first and second orientation indicators 110, 112 enable differentiating between a first end and a second end of the backbone 100. Ultimately, this enables determining the orientation, directionality or polarity of the backbone 100, for example from the first orientation indicator 110 on the first end to the second orientation indicator 112 on the second end.

The backbone 102 is sheet-like, which may be a large linear DNA molecule or an assembly of multiple DNA molecules. Sheet-like backbones may increase the number of available attachment sites substantially. In order to be able to determine the orientation of the backbone 102, a third orientation indicator 114 is provided. Further geometries are possible, for example, the tetrahedral backbone 104, the cubic backbone 106, or the polyhedral backbone 108. These may comprise a fourth orientation indicator 116 in order to determine their orientation.

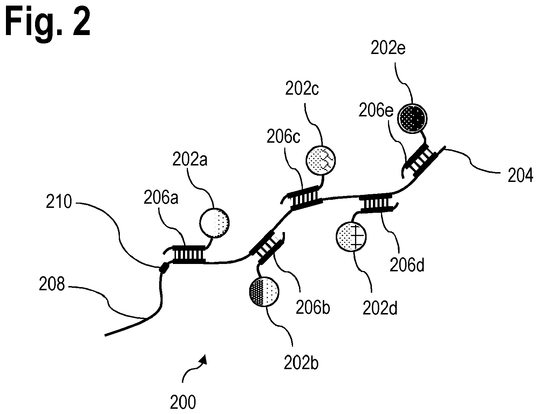

Figure 2 shows schematically a label 200 for attachment to the attachment sites of the backbones 100, 102, 104, 106, 108. The label 200 comprises several fluorescent dyes 202a, 202b, 202c, 202d, 202e. The fluorescent dyes 202a to 202e may differ in their fluorescent properties, for example, excitation wavelengths, emission wavelengths and fluorescent lifetime characteristics. Preferably, the dyes 202a to 202e of the label 200 may be individually identified in a read-out in particular of the label 200. Depending on the number of dyes being used when generating the label 200, a certain number of unique labels can be generated. Typically, the number of different dyes used in total may be in the range of 5-50, for example. For 20 dyes and when using 5 dyes for each label it is easily possible to generate a set of labels with 15,504 unique labels.

The dyes 202a to 202e are individually attached to a label support 204. The label support 204 may be an oligonucleotide and each of the dyes 202a to 202e may be specifically attached to the label support 204 via a unique hybridisation part 206a, 206b, 206c, 206d, 206e. Moreover, the one end 208 of the label support 204 may be specifically attached to the backbones 100 to 108, as described in more detail below. A cleavage site 210 may be provided to cleave the label support 204. This enables removing the dyes 202a to 202e from the end 208 of the label support 204, for example, when the label 200 is attached to one of the backbones 100 to 108. The orientation indicators 110, 112, 114, 116, preferably have the same or a similar structure as described for the label 200.

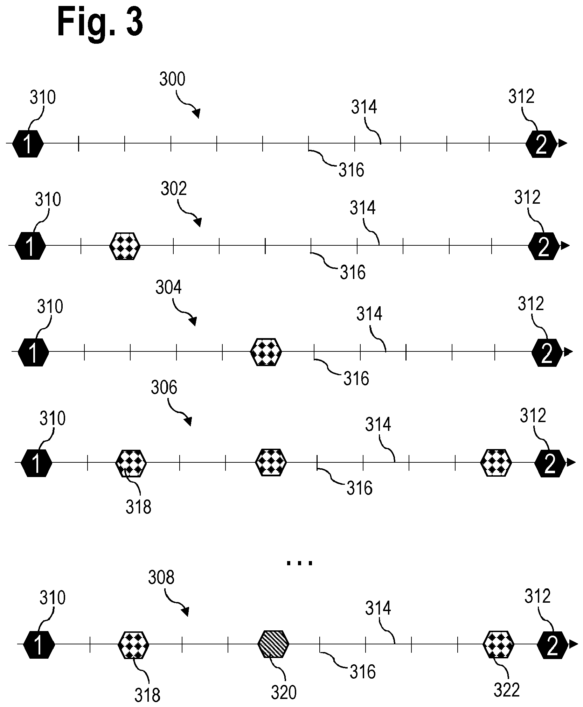

Figure 3 shows schematically a variety of rod-shaped markers 300, 302, 304, 306, 308. The markers 300 to 308 each comprise a first orientation indicator 310 and a second orientation indicator 312, at a first attachment site and a second attachment site of the backbone 314, respectively. In addition, there are ten further attachment sites, one of which is indicated by reference sign 316. In case of the marker 308, labels 318, 320, 322 are attached at three further attachment sites 316. The attachment sites 316 of each backbone 314 is unique such that the labels 318, 320, 322 are specifically attachable to a particular attachment site 316. The markers 300, 302, 304, 306, 308 are preferably between 1 to 2 µm in length.

The orientation indicators 310, 312 generate a relative coordinate system for the markers 300, 302, 304, 306, 308, on which each attachment site 316 may be placed. For example, each attachment site 316 may be assigned an index n with n=1, 2, 3, ..., based on the unique location of the respective attachment site 316. Thus, the different markers 300 to 308 can all be distinguished visually, due to their use of labels with differing properties and/or the labels being attached (or not being attached) at different, distinguishable attachment sites 316 along the backbone 314.

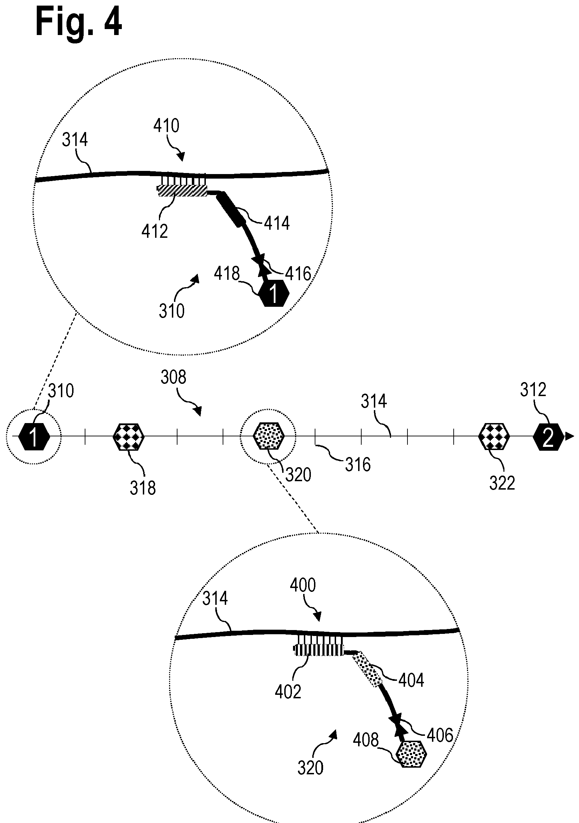

Figure 4 shows schematically details of the rod-shaped marker 308, in particular, the label 320 and its attachment to the backbone 314. The label 320 is attached to the backbone 314 at a particular attachment site 400 of the backbone 314. This attachment site 400 has a unique oligonucleotide sequence that allows hybridisation of a complementary attachment oligonucleotide portion 402 of the label 320. Thus, each attachment site of the marker 308 has a unique oligonucleotide sequence enabling to specifically target labels for each one of the attachment sites.

In addition, the label 320 comprises an encoding oligonucleotide portion 404. The encoding portion 404 is an oligonucleotide sequence that is unique to the fluorescent dye or dyes 408 the label 320. This means that the dyes of a particular label may be identified by the sequence of the encoding portion.

The label 320 also comprises a cleavage site 406 for removing the dye 408 from the encoding portion 404 and the attachment portion 402.

Thus, each label has a unique sequence for the particular dyes (e.g. the encoding portion 404) and a further unique sequence that hybridises to a particular one of the attachment sites of a backbone (e.g. the attachment portion 402).

The first and second orientation indicators 310, 312 are similarly constructed. For example, the orientation indicator 310 is attached to an attachment site 410 of the backbone 314 by a unique complementary attachment oligonucleotide portion 412. Further, the orientation indictor 310 comprises an encoding portion 414, that is unique to the dye or dyes 418 of the orientation indicator. The dyes 418 may be removed from the encoding portion 414 and the attachment portion 412 by cleaving a cleavage site 416.

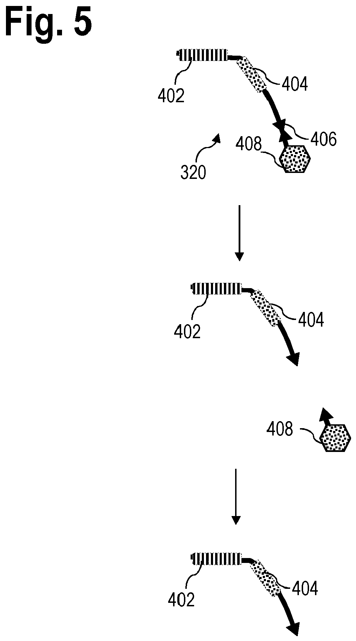

Figure 5 shows steps for preparing the label 320 for sequencing, for example, to read the information of the attachment portion 402 and/or the encoding portion 404. Initially, the label 320 is removed from the backbone e.g. by heating to melt the hybridised attachment portion 402 off the backbone. Next the dyes 408 are removed from the label 320 by cleaving the cleavage site 406, for example, by enzymatic cleavage in case the cleavage site 406 is a restriction site. Alternatively, the cleavage site 406 may be cleaved by light or temperature. The remaining attachment portion 402 and encoding portion 404 may be separated, for example, by chromatography and subsequently sequenced. To that end, universal primers may be ligated to the remaining attachment portion 402 and encoding portion 404. Alternatively, the universal primers may be provided with the label support.

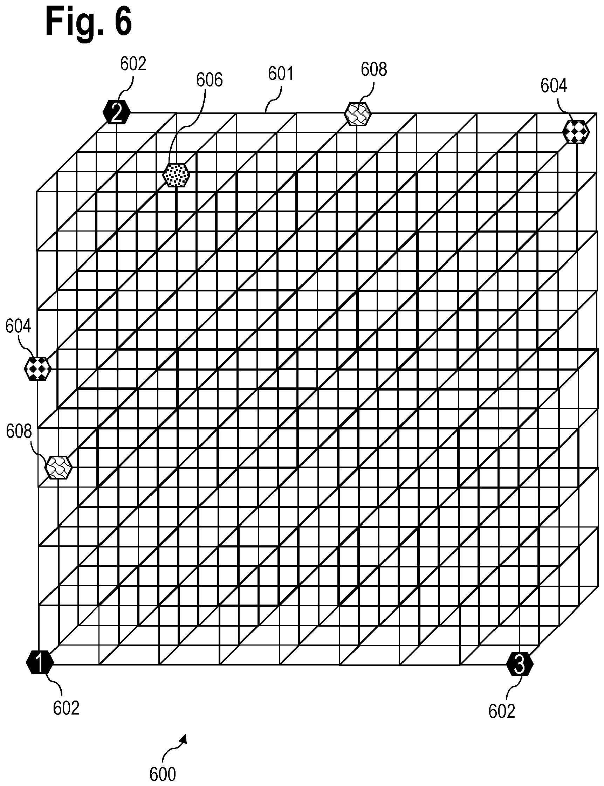

Figure 6 shows schematically an example of a cube-shaped marker 600 with a three-dimensional array backbone 601. The marker 600 comprises three orientation indicators 602. Further, the marker 600 comprises a set of different labels 604, 606, 608. Each label 604, 606, 608 is attached at a particular attachment site of the marker 600. The attachment sites may, for example, be at the corners or edges of the array of the backbone 601. The labels 604, 606, 608 differ in their fluorescent properties, such as fluorescent lifetime, emission wavelength and excitation wavelength.

Thus, similarly to the markers 300 to 308 in Figure 3, the marker 600 may be distinguished from other markers in an optical read-out by the placement of particular labels at specific attachment sites of the backbone 601. In comparison to the rod-shaped markers 300 to 308, this example of the cube-shaped marker 600 provides for around 360 attachment sites for labels, which increases the number of possible combinations of labels and their position on the backbone 601. The physical size of the cube-shaped marker 600 is in the range of 2.5 to 10 µm per side of the cube.

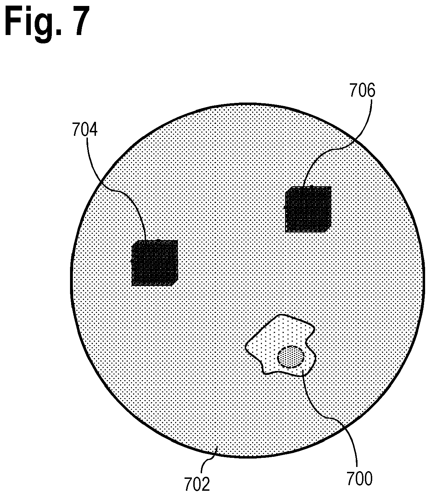

Figure 7 shows schematically a cell 700 embedded in a droplet 702. The droplet 702 may be a hydrogel bead. The droplet 702 further comprises cube-shaped markers 704, 706. The markers 704, 706 may be similar in structure to the marker 600. However, the markers 704, 706 may differ from each other in the specific embodiment of the backbone and/or in the specific labels attached to the respective backbone and/or in the specific attachment sites the labels are attached to.

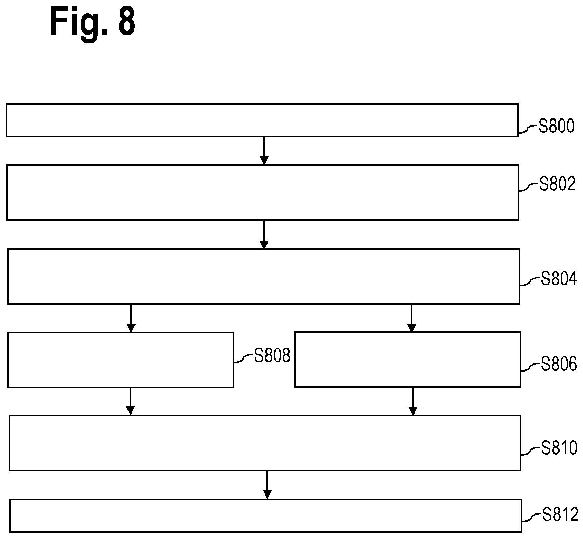

Figure 8 shows a flow chart of an example of a method for sequencing of target genetic sequences of a plurality of cells. The cells are preferably immunological cells, for example, comprising target genetic sequences encoding immunoglobulins.

In a first step S800 of the method, a plurality of droplets with cells and markers is provided. In order to provide or generate the droplets, the cells may individually be embedded in droplets together with at least one marker. Preferably, each droplet embeds an immunological cell with unique target genetic sequences. The droplets may be solid or solidified droplets comprising a hydrogel. These may also be termed hydrogel beads. Alternatively, the droplets may be liquid droplets, for example a water oil emulsion.

The embedding may be performed using a microfluidic device that embeds the markers and the cells as the droplets are generated. Preferably, further cells may be included in the droplet, such as non-immunological cells, that act as a target for the immunological cells, causing the immunological cells to interact with the non-immunological cells. The droplets may be cultured together in the same vessel in a liquid medium after embedding.

In a step S802, the cells, in particular the immunological cells, in each droplet are lysed in order to release their genetic content, in particular the target genetic sequences. Optionally, the respective of the markers, in particular the oligonucleotides of the labels, may be released from the backbone of the markers.

In step S804, the target genetic sequences and the oligonucleotides may be amplified by polymerase chain reaction (PCR). To this end, suitable primers are introduced into the droplets. This results in a plurality of copies of each of the labels' oligonucleotides and the target genetic sequences. The primers may comprise restriction sites to be introduced into the amplification products.

In step S806, the amplified target genetic sequences and the oligonucleotides of the labels are ligated together to generate sequencing constructs. This may be achieved by cutting the amplification products of step S804 with restriction enzymes suitable for the restriction sites introduced in step S804. Thus, the generated sticky ends may enable ligating the amplification products to each other. For example, each target genetic sequence and each oligonucleotide of a label may have a complementary restriction site, such that one target genetic sequence randomly binds to an oligonucleotide of a label. To ensure that a particular target genetic sequence of a droplet may be unambiguously correlated to the respective marker of the droplet, a representative number of random sequencing constructs have to be generated and sequenced. This ensure, that each combination of unique label oligonucleotide ligated to a copy of the target genetic sequence is among the random sequencing constructs. Alternatively to step S806, the sequencing constructs may be generated from the amplified target genetic sequences and the oligonucleotides of the labels in step S808. In step S808 the sequencing constructs are generated, such that all oligonucleotides of the labels of a marker of a particular droplet and the target genetic sequences are ligated together. Specifically, the ligation order may be predetermined. For example, certain restriction sites may be introduced during amplification in step S804 that allow combining all oligonucleotides of the labels and the target genetic sequences of a droplet in a certain order. This ensures that each predetermined sequencing construct carries all the encoding and attachment portions of the labels of a particular droplet together with the target genetic sequences. This enables unambiguous correlating a particular target genetic sequence to the respective marker of the droplet based on the combination of encoding and attachment portions in each predetermined sequencing construct.

In step S810 the sequencing constructs are sequenced in order to generate sequencing data of the sequencing constructs. Predetermined sequencing constructs generated in step S808 may be pooled for all droplets, since each sequencing construct carries the unique combination of encoding and attachment portions of the labels of a particular droplet together with the target genetic sequences. In contrast the random sequencing construct generated in step S806 need to be sequenced for each droplet individually, since the random sequencing constructs only carry the unique combination of encoding and attachment portions of the labels of a particular droplet only collectively, but not individually. The method ends in step S814.

The method may comprise further steps. For example, in an additional step before step S804, an optical readout, preferably an image or an image stack, e.g. generated with a microscope, of the cells together with the markers of each droplet is generated. The optical readout may be analysed to determine the markers associated with each droplet. The markers may each be identified by the particular labels attached to the particular attachment sites. This includes the fluorescent properties of the dyes of the labels.

In an additional step following step S812, the presence of the sequence of the attachment oligonucleotide portions and the sequence of the encoding portions is determined in the sequencing data. This enables determining the presence of respective markers. The sequencing data is correlated to the cells of a particular droplet based on the presence of the sequences and the individual markers present in the droplet. For each marker it is known which labels are attached at which attachment sites, therefore, in the optical readout, the markers present may be identified unambiguously. In the sequencing the identity of the markers present in the sequenced sample can be likewise identified by determining the presence of the respective encoding and attachment oligonucleotide sequences. By comparing these, an optical readout of a droplet with particular markers may be correlated or assigned to sequencing data of that droplet containing these particular markers. This enables directly linking data about the phenotype in the optical readout with data about the genotype in the sequencing data.

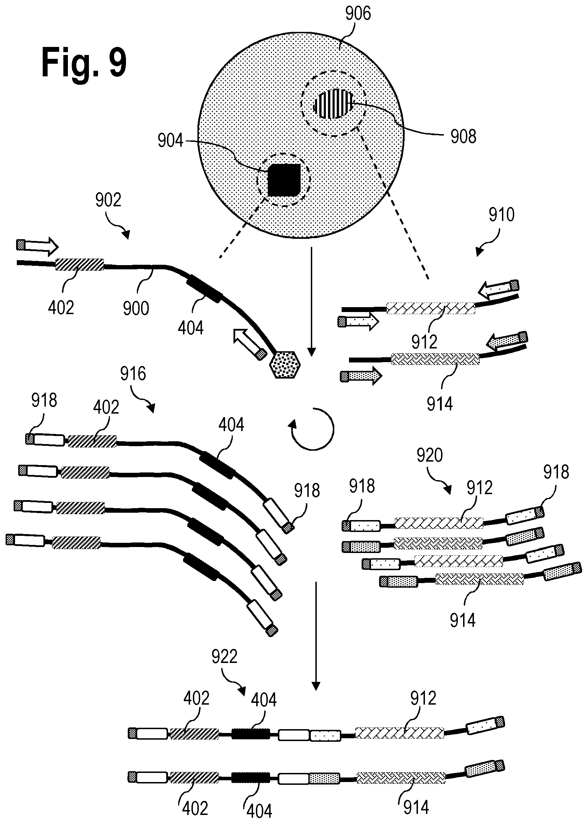

Figure 9 shows details of steps to generate random sequencing constructs 922. An oligonucleotide 900 of a label 902 of a marker 904 is embedded in a droplet 906 together with a cell 908. The oligonucleotide 900 may be amplified together with target genetic sequences 910 by PCR. The oligonucleotide 900 comprises the attachment portion 402 and the encoding portion 404. The target genetic sequences 910 may comprise several unique sequences 912, 914 of interest. Similarly, the marker 904 may comprise several unique labels 902, for simplicity only a single unique label 902 is shown in Figure 9.

Amplified labels 916 may include restriction sites 918 introduced by primers used for amplification. Similarly, amplified target genetic sequences 920 may include the restriction sites 918. Upon digestion with the respective restriction enzymes, the amplified labels 916 and amplified target genetic sequences 920 may be ligated to form random sequencing constructs 922. These are shown in Figure 9 exemplarily as combinations of one of the amplified labels 916 and the amplified target genetic sequences 920. In case the marker 904 comprises a plurality of unique labels 902, the random sequencing constructs 922 may comprise a plurality of combinations of the respective amplified labels and the amplified target genetic sequences 920.

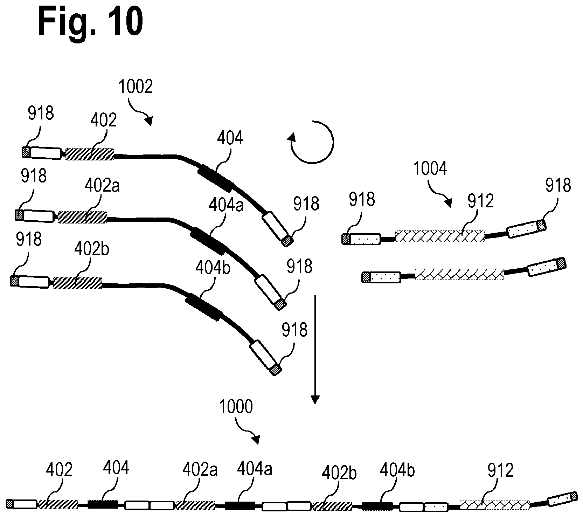

Figure 10 shows details of steps to generate predetermined sequencing constructs 1000. In contrast to Figure 9, the amplified labels 1002 are generated from a marker comprising a plurality of unique labels. Thus, the amplified labels 1002 comprise a plurality of unique attachment portions 402, 402a, 402b and a plurality of unique encoding portions 404, 404a, 404b. Thus, the amplified labels 1002 may be used to unambiguously identify the respective marker based on the combination of the portions 402, 402a, 402b, 404, 404a, 404b.

Amplified target genetic sequences 1004 are exemplarily shown as copies with the unique sequence 912. In order to generate the predetermined sequencing construct 1000, a copy of each of the amplified labels 1002 is ligated with a copy of the amplified target genetic sequences 1004. In particular, this may be achieved by choosing restriction sites 918 for amplification such that the parts of the predetermined sequencing construct are ligated in a predetermined order.

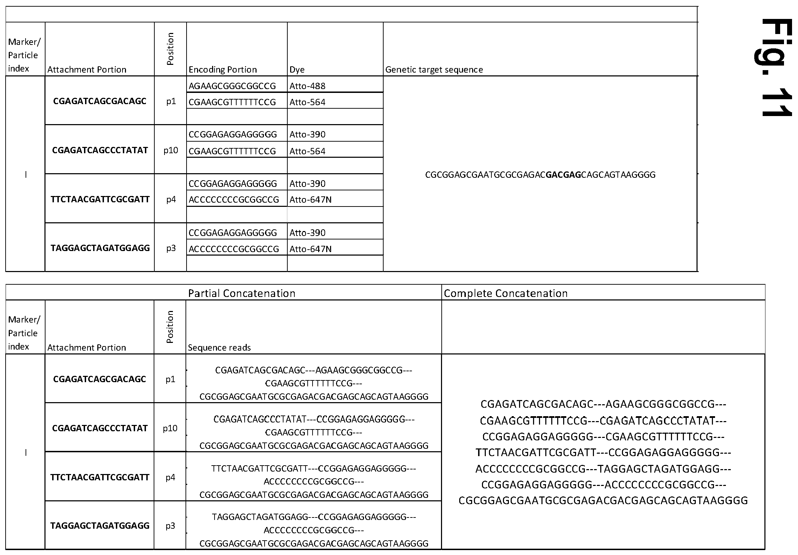

Figure 11 shows a hypothetical example of a database entry for an optically detectable marker I, which was incorporated into a given droplet or particle alongside a single cell/clone and used to assign the particle index I to this particular droplet or particle and contained single cell/clone. In this example the optically detectable marker had combinatorial labels, comprising two dyes each, attached to positions p1, p10, p4, p3, which are addressed via the corresponding attachment oligonucleotide portion. Each dye being connected to the label via an oligonucleotide, which has at least the encoding oligonucleotide portion. The dye species can therefore be determined optically or by reading/sequencing the encoding oligonucleotide portion. The position of said dye can be determined optically in relation to orientation markers or by reading the attachment oligonucleotide portion, which is physically connected to the encoding oligonucleotide portion or a complementary sequence, depending on the way the label is being designed. Further as shown in Figure 11 clone I in droplet I marked with marker I had the target genetic sequence shown in the table. This target genetic sequence may be concatenated or ligated to a part or the full complement of attachment-encoding oligonucleotide portions.

In the sense of this document reading or sequencing the attachment oligonucleotide portion or encoding oligonucleotide portion may be performed on the sense strand or complimentary antisense strand. So in the sense of this document both the sense and antisense complimentary of either attachment oligonucleotide portion or encoding oligonucleotide portion is meant, as both are equally suited for identifying a particular position on the marker or a particular dye of the marker.

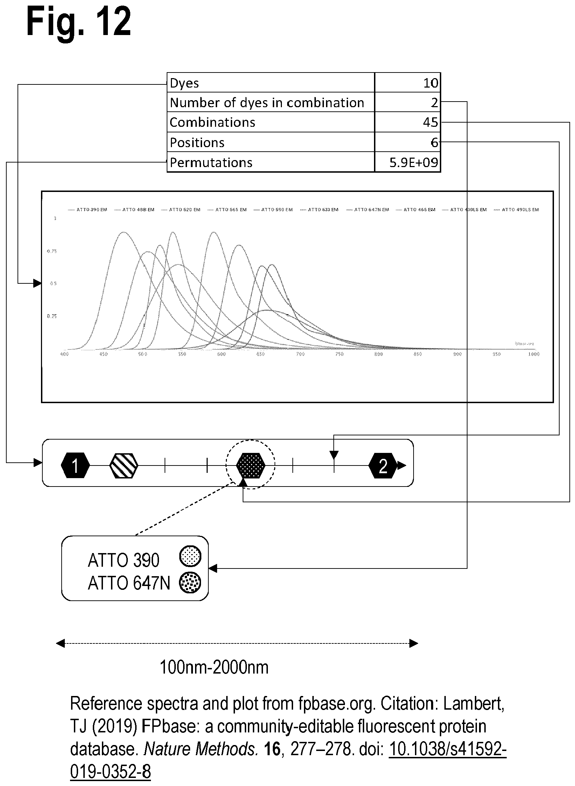

Figure 12 provides another example, wherein a microscopic readout is used, that can differentiate a panel of 10 ATTO dyes, wherein unique combinatorial labels are generated that contain 2 out of these 10 dyes, leading to 45 unique combinations. A backbone is used with 6 positions and two orientation markers, that are spaced apart around 200nm on a rod-like structure of about 1600nm length. The combination of two-layers of encoding in the properties of the dyes (e.g. spectral and life-time) and the spatial encoding on the nanostructure allows to generate a very large number of different markers or codes. This means that simple, rod-like structures in the micron range can be used in conjunction with a limited number of fluorescent dyes to encode billions of particles.

Identical or similarly acting elements are designated with the same reference signs in all Figures. As used herein the term "and/or" includes any and all combinations of one or more of the associated listed items and may be abbreviated as "/". Individual features of the embodiments and all combinations of individual features of the embodiments among each other as well as in combination with individual features or feature groups of the preceding description and/or claims are considered disclosed.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100, 102, 104, 106, 108, 314, 601: Nucleic acid backbone
- 110, 112, 114, 116, 310, 312, 602: Orientation indicator
- 200, 318, 320, 322, 604, 606, 608,902: Label
- 202a, 202b, 202c, 202d, 202e, 408, 418: Fluorescent dye
- 204: Label support
- 206a, 206b, 206c, 206d, 206e: Hybridisation part
- 208: End of label support
- 210, 406, 416: Cleavage site
- 300, 302, 304, 306, 308, 600, 704, 706, 904: Marker
- 316, 400, 410: Attachment site
- 402, 402a, 402b, 412: Attachment oligonucleotide portion
- 404, 404a, 404b, 414: Encoding oligonucleotide portion
- 700,908: Cell
- 702, 906: Droplet
- 900: Oligonucleotide of label
- 910: Target genetic sequence
- 912, 914: Unique sequence of target genetic sequence
- 916, 1002: Amplified labels
- 918: Restriction site
- 920, 1004: Amplified target genetic sequence
- 922: Random sequencing construct
- 1000: Predetermined sequencing construct

## Claims

1. A method for sequencing of target genetic sequences (910) of a plurality of cells (700, 908) comprising the following steps:
providing droplets (702, 906), each droplet including at least one cell (700, 908) of the plurality of cells and at least one optically detectable marker (300, 302, 304, 306, 308, 600, 704, 706, 904) comprising:
a nucleic acid backbone (100, 102, 104, 106, 108, 314, 601) with a plurality of attachment sites (316, 400, 410) at predetermined positions,
a plurality of labels (200, 318, 320, 322, 604, 606, 608, 902) for attachment to at least some of the attachment sites (316, 400, 410),
at least a first orientation indicator and a second orientation indicator,
wherein each label (200, 318, 320, 322, 604, 606, 608, 902) comprises at least one dye (202a, 202b, 202c, 202d, 202e, 408, 418), an encoding oligonucleotide portion (404, 414) configured to encode characteristics of the at least one dye (202a, 202b, 202c, 202d, 202e, 408, 418), and an attachment oligonucleotide portion (402, 412) configured to reversibly attach to one of the attachment sites (316, 400, 410), and
wherein the attachment oligonucleotide portion (402, 412) of each label (200, 318, 320, 322, 604, 606, 608, 902) comprises a unique oligonucleotide sequence configured to bind to a complementary sequence of one of the attachment sites (316, 400, 410),
releasing the target genetic sequences (910) from the cells (700, 908),
ligating the target genetic sequences (910) to the labels (200, 318, 320, 322, 604, 606, 608, 902) of the marker (300, 302, 304, 306, 308, 600, 704, 706, 904) to generate sequencing constructs (922, 1000), and
sequencing the sequencing constructs (922, 1000).

2. The method according to claim 1, wherein the labels (200, 318, 320, 322, 604, 606, 608, 902) are released from the nucleic acid backbone (100, 102, 104, 106, 108, 314, 601) of the marker (300, 302, 304, 306, 308, 600, 704, 706, 904) prior to sequencing.

3. The method according to one of the preceding claims, wherein the target genetic sequences (910) and the oligonucleotide (900) portions of the labels (200, 318, 320, 322, 604, 606, 608, 902) are amplified prior to sequencing.

4. The method according to one of the preceding claims, wherein the label (200, 318, 320, 322, 604, 606, 608, 902) of the marker (300, 302, 304, 306, 308, 600, 704, 706, 904) comprises a cleavage site (210, 406, 416) configured to separate the dye (202a, 202b, 202c, 202d, 202e, 408, 418) from the oligonucleotide (900) of the label (200, 318, 320, 322, 604, 606, 608, 902) and wherein prior to ligating, the dye (202a, 202b, 202c, 202d, 202e, 408, 418) of each label (200, 318, 320, 322, 604, 606, 608, 902) is cleaved off the oligonucleotide (900) of the label at the cleavage site (210, 406, 416).

5. The method according to one of the preceding claims, wherein the droplet (702, 906) is a liquid droplet of a first liquid in an immiscible second liquid or a solid droplet.

6. The method according to one of the preceding claims, wherein the step of providing droplets (702, 906) includes embedding cells (700, 908) and markers (300, 302, 304, 306, 308, 600, 704, 706, 904) in droplets (702, 906), preferably by means of a microfluidic device.

7. The method according to one of the preceding claims, wherein the cells (700, 908) are cultured in the droplets (702, 906).

8. The method according to one of the preceding claims, wherein an optical readout of at least one of the droplets (702, 906) with the at least one cell (700, 908) and the at least one marker (300, 302, 304, 306, 308, 600, 704, 706, 904) is acquired.

9. The method according to claim 8, wherein in the optical readout of the at least one of the droplets (702, 906), the at least one marker (300, 302, 304, 306, 308, 600, 704, 706, 904) in the droplet (702, 906) is determined based on the at least one dye (202a, 202b, 202c, 202d, 202e, 408, 418) of each of the labels (200, 318, 320, 322, 604, 606, 608, 902) of the marker (300, 302, 304, 306, 308, 600, 704, 706, 904).

10. The method according to one of the claims 8 or 9, wherein in sequencing data generated during sequencing of the sequencing constructs (922, 1000) of the at least one of the droplets (702, 906) the sequences of the attachment oligonucleotide portion (402, 412) and the encoding oligonucleotide portion (404, 414) are determined and wherein the sequencing data of the at least one droplet (702, 906) is correlated with the optical readout of the at least one droplet (702, 906) based on the presence of the sequences in the sequencing data and the marker (300, 302, 304, 306, 308, 600, 704, 706, 904) present in the at least one droplet (702, 906).

11. The method according to one of the preceding claims, wherein the target genetic sequences (910) are generated by reverse transcription of mRNA of the at least one cell (700, 908).

12. The method according to one of the preceding claims, wherein the plurality of cells (700, 908) are immunological cells and wherein the target genetic sequences (910) are immunoglobulin genetic sequences.

13. The method according to claim 12, wherein each droplet (702, 906) further comprises at least one non-immunological cell and wherein the at least one immunological cell is specific to antigens of the non-immunological cell.

14. The method according to one of the preceding claims, wherein the step of ligating includes ligating together all target genetic sequences (910) of the cell (700, 908) of one of the droplets (702, 906) to the labels (200, 318, 320, 322, 604, 606, 608, 902) of the marker (300, 302, 304, 306, 308, 600, 704, 706, 904) of one of the droplets (702, 906) to generate the sequencing constructs (922, 1000).

15. The method according to claim 14, wherein the sequencing constructs (922, 1000) of all droplets (702, 906) are pooled and sequenced together.
